# FASCICULE DE BREVET EUROPEEN

(11) **EP 2 322 136 B1**
(45) Date de publication et mention de la délivrance du brevet: **04.07.2012**
(21) Numéro de dépôt: 10190265.8
(22) Date de dépôt: 05.11.2010
(51) Int. Cl.: A61K 8/04, A61K 8/41, A61K 8/73, A61K 8/81, A61Q 5/12

(54) **Composition cosmétique pour le conditionnement des cheveux contenant un mélange de particules creuses, d'amidon et de tensioactif cationique**
Kosmetische Zusammensetzung für die Behandlung von Haaren, die eine Mischung aus Hohlpartikeln, Stärke und kationischen Tensiden enthält
Cosmetic hair-conditioner composition containing a mixture of hollow particles, starch and a cationic surfactant

(30) Priorité: 13.11.2009 FR 0958026
(43) Date de publication de la demande: 18.05.2011
(73) Titulaire: L'Oréal, 75008 Paris (FR)
(72) Inventeur: Bourdin, Claire, 92300, Levallois Perret (FR); Sellier, Céline, 94140, Alfortville (FR); Gabin, Gérard, 75009, Paris (FR)
(74) Mandataire: Dossmann, Gérard

(56) Documents cités:
- EP-A1- 0 692 248
- EP-A2- 1 698 326
- FR-A1- 2 779 648
- FR-A1- 2 824 733

## Description

La présente invention se rapporte à une composition cosmétique se présentant sous forme de solide déformable comprenant des particules creuses, un amidon modifié ou non et un tensioactif cationique, dans un rapport amidon/tensioactif cationique particulier.

De nombreux produits de soin capillaire ont été décrits dans l'art antérieur. Les compositions correspondantes ont pour but d'apporter de bonnes propriétés cosmétiques aux cheveux.

Les produits classiques de soins du cheveu, par exemple les après-shampooing ou les masques, se présentent le plus souvent sous forme de crèmes plus ou moins visqueuses. Ces produits fluides sont difficiles à doser, notamment parce qu'ils ont tendance à s'échapper entre les doigts ou à s'échapper de leur conditionnement, ce qui peut être très gênant lorsqu'ils viennent au contact des vêtements, par exemple lors de déplacements.

Pour modifier la texture, et notamment la rendre plus compacte, les moyens classiques consistent à utiliser des épaississants ou à augmenter le taux de phase grasse. Mais cela se fait souvent au détriment des effets cosmétiques de la composition.

Par ailleurs, les compositions conditionnantes les plus épaisses présentent souvent l'inconvénient de nécessiter beaucoup d'eau de rinçage afin d'éliminer le surplus de produit sur les cheveux. Dans de nombreux pays où l'accès à l'eau est restreint, le temps de rinçage et donc la quantité nécessaire pour bien rincer le produit sont des indicateurs clés des qualités d'usage d'une composition.

En outre, les compositions de soin du cheveu, qu'il s'agisse de produits à rincer ou non, sont en grande majorité constitués de suspensions de cristaux de corps gras. Ces suspensions ne permettent pas toujours d'incorporer les matières premières souhaitées ou au taux voulu. En effet une telle incorporation peut parfois entraîner des phénomènes de déstabilisation.

Enfin, les utilisateurs recherchent de plus en plus de nouvelles textures et de nouveaux types de produits.

Il existe donc un besoin de disposer de compositions de soin du cheveu qui ne coulent pas et qui soient plus compactes, qui nécessitent peu d'eau pour le rinçage et qui permettent d'éviter les phénomènes d'agrégation des cristaux et donc les floculations. Les compositions recherchées doivent être d'une application aisée sur les cheveux, d'une grande légèreté et d'une grande douceur.

La demanderesse a découvert de manière surprenante qu'une composition comprenant l'association de particules creuses, d'amidon et de tensioactif cationique permettait de résoudre les problèmes de l'art antérieur.

L'invention a donc pour objet une composition cosmétique se présentant sous forme de solide déformable comprenant, dans un milieu cosmétiquement acceptable, un ou plusieurs types de particules creuses, un ou plusieurs amidons modifiés ou non, et un ou plusieurs tensioactifs cationiques, le rapport pondéral tensioactif(s) cationique(s)/amidon(s) étant supérieur ou égal à 0,3.

Par composition se présentant sous forme de solide déformable, on entend au sens de la présente invention une composition qui se présente sous une forme solide, sèche, malléable, ressemblant à de la guimauve (voir le document US-A-3 682 659 pour la consistance de la guimauve).

La densité de la composition selon l'invention est avantageusement comprise entre 0,2 et 0,6 et plus précisément entre 0,4 et 0,5.

L'un des avantages de la composition est qu'elle ne risque pas de s'échapper de son conditionnement notamment lors de son transport. Par ailleurs, cette composition est très facilement préhensible et ne s'écoule pas entre les doigts. Son dosage est beaucoup plus simple que celui des compositions liquides.

La composition selon l'invention a encore comme avantage de présenter de bonnes propriétés de fondant lors de l'application. Les cheveux sont souples lors de l'application. Elle conduit également à des propriétés de souplesse des cheveux au rinçage, de démêlage et de souplesse sur cheveux humides, et de lissage sur cheveux secs. En outre, cette composition présente un bon délitement et conduit à une texture onctueuse après transformation en crème dans la main.

Un autre avantage de cette composition est un excellent confort à l'application. En particulier, on ne constate aucune coulure de la composition, contrairement aux compositions classiques, risquant d'irriter notamment le visage et les yeux. L'absence de coulure est très appréciée dans le cas des permanentes et colorations, ainsi que pour les shampooings destinés aux enfants.

Selon l'invention, la composition se présente donc sous l'aspect d'un solide déformable ne tâchant pas et ressemblant à de la guimauve. Ce solide peut être modelé comme de la pâte à modeler pour enfants. Il peut être rompu facilement à la main afin de ne prélever que la quantité nécessaire de produit. En particulier, cette composition peut être conditionnée sous forme de mono dose et par exemple sous forme de petits cubes ou de berlingots. Ce conditionnement sous forme de mono doses est particulièrement avantageux d'un point de vue bactériologique dans le sens où le produit étant totalement consommé en une seule application, il n'existe pas de reliquat de produit susceptible de se polluer après le contact avec les mains et/ou l'air ambiant. Ceci permet de mettre en oeuvre des systèmes conservateurs moins concentrés.

Grâce aux particules de l'invention, il est notamment possible d'obtenir une structure homogène (solide déformable) pour des constituants conduisant normalement à des phases distinctes (constituants non miscibles par exemple huile, eau).

Les particules creuses présentes dans la composition selon l'invention sont avantageusement choisies parmi les particules de verre, les particules de matériaux thermoplastiques choisis parmi les polyamides tels que le Nylon, les polymères ou copolymères d'acrylonitrile, de chlorure de vinylidène, de chlorure de vinyle et/ou de monomère acrylique ou styrénique, éventuellement expansés, les microsphères microporeuses et les microparticules de silice.

Le monomère acrylique est par exemple un acrylate ou méthacrylate de méthyle ou d'éthyle. Le monomère styrénique est par exemple l'a-méthyl-styrène ou le styrène.

Comme particules de verre utilisables dans l'invention, on peut citer les billes de verre creuses vendues par la société 3M sous la référence Scotchlite Glass Bubbles S 22. 95% de ces billes ont un diamètre inférieur à 74 µm.

De préférence, les particules sont des particules creuses déformables d'un copolymère expansé de chlorure de vinylidène et d'acrylonitrile, ou de chlorure de vinylidène, d'acrylonitrile et de (méth)acrylate ou monomère styrénique. On peut par exemple utiliser un polymère contenant 0-60% de motifs dérivés du chlorure de vinylidène, 20-90% de motifs dérivés d'acrylonitrile et 0-50% de motifs dérivés d'un monomère acrylique ou styrénique, la somme des pourcentages (en poids) étant égale à 100%. Le monomère acrylique peut être le (méth)acrylate de méthyle ou d'éthyle. Le monomère styrénique peut être le styrène ou le α-méthyl-styrène.

Plus préférentiellement, les particules utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.

Les particules utilisables dans l'invention sont par exemple les microsphères de terpolymère expansé de chlorure de vinylidène, d'acrylonitrile et de méthacrylate de méthyle, vendues sous la marque EXPANCEL par la société Nobel Casco et en particulier sous les références 551 DE 12 (granulométrie D(0,5) d'environ 12 µm et masse volumique d'environ 40 kg/m³), 551 DE 20 (granulométrie D(0,5) d'environ 15 à 25 µm et masse volumique d'environ 60 kg/m³), 551 DE 50 (granulométrie D(0,5) d'environ 40 µm), 461 DE 50 et 642 WE 50 de 50 µm de granulométrie D(0,5) environ, 551 DE 80 (granulométrie D(0,5) de 50 à 80 µm environ). On peut aussi utiliser des particules de ce même terpolymère expansé, ayant une granulométrie D(0,5) d'environ 18 µm et une masse volumique d'environ 60 à 80 kg/m³ (Expancel EL23) ou encore de granulométrie D(0,5) d'environ 34 µm et de masse volumique d'environ 20 kg/m³. On peut encore citer les particules EXPANCEL 551 DE 40 d42 (granulométrie D(0,5) d'environ 30 à 50 µm et masse volumique d'environ 42 kg/m³), 551 DE 80 d42 (granulométrie D(0,5) d'environ 50 à 80 µm et masse volumique d'environ 42 kg/m³), 461 DE 20 d70 (granulométrie D(0,5) d'environ 15 à 25 µm et masse volumique d'environ 70 kg/m³), 461 DE 40 d25 (granulométrie D(0,5) d'environ 35 à 55 µm et masse volumique d'environ 25 kg/m³), 461 DE 40 d60 (granulométrie D(0,5) d'environ 20 à 40 µm et masse volumique d'environ 60 kg/m³), 461 DET 40 d25 (granulométrie D(0,5) d'environ 35 à 55 µm et masse volumique d'environ 25 kg/m³), 051 DE 40 d60 (granulométrie D(0,5) d'environ 20 à 40 µm et masse volumique d'environ 60 kg/m³), 091 DE 40 d30 (granulométrie D(0,5) d'environ 35 à 55 µm et masse volumique d'environ 30 kg/m³), 091 DE 80 d30 (granulométrie D(0,5) d'environ 60 à 90 µm et masse volumique d'environ 30 kg/m³). On peut encore utiliser des particules de polymère de chlorure de vinylidène et d'acrylonitrile ou de chlorure de vinylidène, d'acrylonitrile et de méthacrylate de méthyle non expansé comme celles vendues sous la marque EXPANCEL avec la référence 551 DU 10 (granulométrie D(0,5) d'environ 10 µm) ou 461 DU 15 (granulométrie D(0,5) d'environ 15 µm).

Parmi les microsphères microporeuses utilisables dans la composition selon l'invention, on peut citer celles vendues par Dow Corning sous la dénomination « POLYTRAP » qui sont formées de copolymères méthacrylate de lauryle/diméthacrylate d'éthylèneglycol ; ou celles vendues par Seppic sous la dénomination « MICROPEARL ».

Parmi les microparticules de silice utilisables dans la composition selon l'invention, on peut citer les microparticules de silice creuses telles que celles vendues par Miyoshi Kasei sous la dénomination « SILICA BEADS S700 ».

Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle.

De façon préférentielle, la masse volumique des particules creuses utilisables dans la composition selon l'invention est choisie dans la gamme allant de 15 à 200 kg/m³ et mieux de 20 à 120 kg/m³, et encore mieux de 30 à 80 kg/m³.

Les particules creuses présentes dans la composition selon l'invention ont généralement une granulométrie moyenne D(0,5) variant de 1 µm à 300 µm, par exemple de 5 µm à 200 µm, de préférence de 10 µm à 100 µm, mieux de 15 µm à 50 µm.

De préférence, les particules creuses présentes dans la composition selon l'invention sont remplies d'un gaz. Ce gaz peut être de l'air, de l'azote, de l'isobutane ou de l'isopentane.

Les particules creuses de l'invention sont de préférence des microsphères de terpolymère expansé de chlorure de vinylidène, d'acrylonitrile et de méthacrylate de méthyle.

Les particules creuses présentes dans la composition selon l'invention représentent généralement de 0,01 à 15%, de préférence de 1 à 10%, en poids du poids total de la composition.

Comme expliqué précédemment, la composition selon l'invention comprend un ou plusieurs amidons modifiés ou non.

Le ou les amidons utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine végétale des amidons.

Les molécules d'amidons utilisés dans la présente invention peuvent provenir d'une source végétale telle que les céréales, les tubercules, les racines, les légumes et les fruits. Ainsi, le ou les amidons peuvent provenir d'une source végétale choisie parmi le maïs, les pois, la pomme de terre, la patate douce, la banane, l'orge, le blé, le riz, l'avoine, le sagou, le tapioca et le sorgo. L'amidon est de préférence issu de la pomme de terre.

On peut également utiliser les hydrolysats des amidons cités ci-dessus.

Les amidons se présentent généralement sous la forme d'une poudre blanche, insoluble dans l'eau froide, dont la taille des particules élémentaires va de 3 à 100 microns.

Les amidons utilisés dans la composition de l'invention peuvent être modifiés chimiquement par une ou plusieurs des réactions suivantes : prégélatinisation, oxydation, réticulation, estérification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glycéryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyalkyl (en particulier carboxyméthyl), octénylsuccinique. On peut citer en particulier les amidons modifiés par le carboxyméthyl de sodium.

On peut notamment obtenir par réticulation avec des composés phosphorés des phosphates de monoamidon (du type Am-O-PO-(OX)2), des phosphates de diamidon ( du type Am-O-PO-(OX)-O-Am) ou même de triamidon (du type Am-O-PO- (O-Am)2) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon, en particulier hydroxypropylé, ou des composés riches en phosphate de diamidon, en particulier hydroxypropylé, comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs hydroxypropylé gélatinisé).

Lorsque les amidons sont modifiés chimiquement par une réaction d'estérification, on peut obtenir des carboxyalkylamidons, comme indiqué précédemment.

Les carboxyalkylamidons sont de préférence des carboxyalkyl (C₁-C₄)_{_}amidon et plus particulièrement des carboxyméthylamidons.

Les sels sont notamment des sels de métal alcalin ou alcalinoterreux tels que Na, K 1/2, Li, NH₄, d'un ammonium quaternaire ou d'une amine organique telle que la mono, di ou triéthanolamine.

Les carboxyalkylamidons sont obtenus par greffage de groupements carboxyalkyl sur une ou plusieurs fonctions alcools de l'amidon, notamment par réaction d'amidon et de monochloroacétate de sodium en milieu alcalin.

Les groupements carboxyalkyl sont généralement fixés par l'intermédiaire d'une fonction éther, plus particulièrement sur le carbone 1.

Le degré de substitution va de préférence de 0,1 à 1 et plus particulièrement de 0,15 à 0,5. Le degré de substitution est défini selon la présente invention comme étant le nombre moyen de groupements hydroxyles substitués par un groupement ester ou éther (en l'occurrence éther pour les carboxyméthylamidons) par unité monosaccharidique du polysaccharide.

Les carboxyalkylamidons comprennent de préférence des motifs de formule suivante:

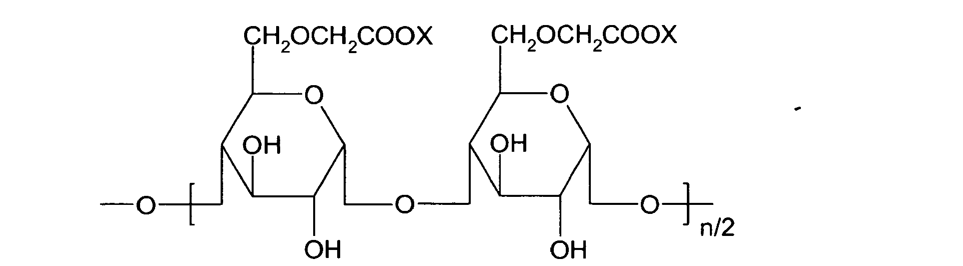

X désigne un atome d'hydrogène, un métal alcalin ou alcalinoterreux tels que Na, K 1/2, Li, NH₄, un ammonium quaternaire ou une amine organique. De préférence X désigne un ion Na+.

Les carboxyalkylamidons utilisables selon la présente invention sont de préférence les carboxyalkylamidons non prégélatinisés.

Les carboxyalkylamidons utilisables selon la présente invention sont de préférence les carboxyalkylamidons réticulés partiellement ou totalement.

Les carboxyalkylamidons utilisables selon la présente invention sont de préférence des sels de sodium de carboxyalkylamidons, en particulier un sel de sodium de carboxyméthylamidon de pomme de terre vendus notamment sous la dénomination PRIMOJEL par la société DMV International. Plus de 95% des particules de cet amidon ont un diamètre inférieur à 100 microns et plus particulièrement inférieur à 65 microns.

Selon l'invention, on peut aussi utiliser des amidons amphotères, ces amidons amphotères contiennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 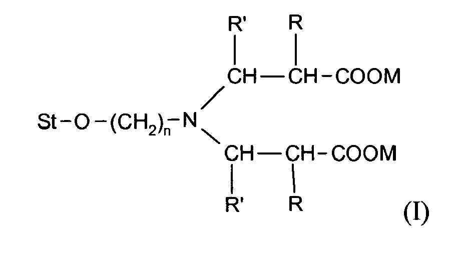 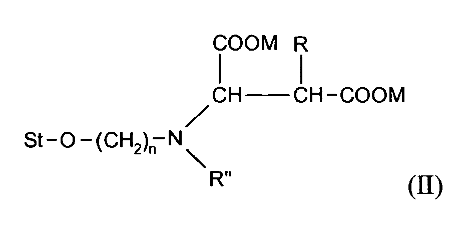 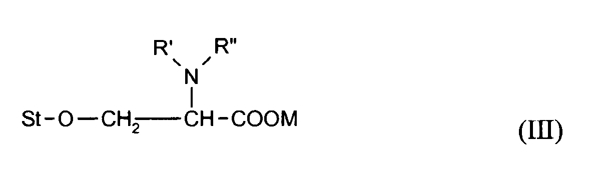 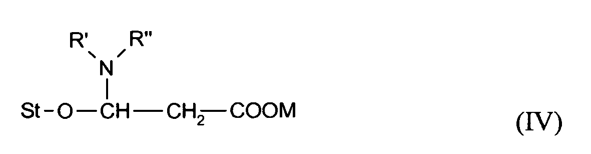 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" et M représentent un atome d'hydrogène et n est égal à 2. On peut citer en particulier la fécule de pomme de terre modifiée par de l'acide 2-chloroéthyl aminodipropionique neutralisée à la soude, commercialisée sous la référence STRUCTURE SOLANACE par la société NATIONAL STARCH.

De préférence le ou les amidons utilisés dans l'invention sont chimiquement modifiés.

Le ou les amidons présents dans la composition selon l'invention représentent généralement de 0,01 à 15%, de préférence de 0,1 à 10 mieux de 1 à 10%, en poids du poids total de la composition.

Comme expliqué précédemment, la composition selon l'invention comprend également un ou plusieurs tensioactifs cationiques.

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

On peut citer en particulier le méthosulfate de méthyl alkyl alkylamidoéthyl imidazolinium (Quaternium-87) commercialisé par la société DEGUSSA sous la référence VARISOFT W 575 PG, ou le chlorure de béhényl triméthyl ammonium commercialisé par la société CLARIANT sous la référence GENAMIN KDMP. On peut encore citer le chlorure de cétyl triméthyl ammonium.

On peut aussi utiliser à titre de tensioactifs cationiques les sels d'ammonium quaternaire contenant au moins une fonction ester tels que ceux de formule (V) suivante : 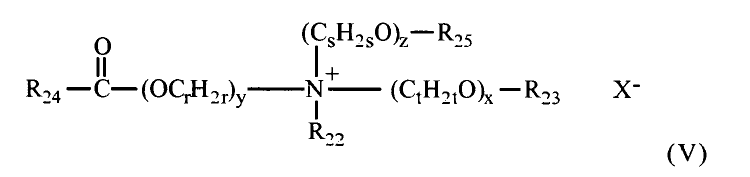 dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 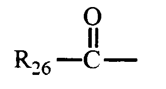
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 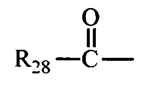
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6;
y est un entier valant de 1 à 10 ;
x et z, identiques ou différents, sont des entiers valant de 0 à 10 ;
X⁻ est un anion simple ou complexe, organique ou inorganique;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

Parmi les sels d'ammonium de formule (V), on utilise plus particulièrement les composés dans lesquels :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
- le radical 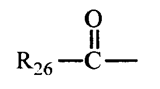
- les radicaux méthyle, éthyle ou hydrocarbonés en C₁₄-C₂₂,
- l'atome d'hydrogène ;
- R₂₅ est choisi parmi :
- le radical 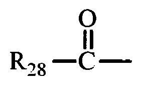
- l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (V) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents. On peut citer en particulier le méthosulfate de distéaroyléthyl hydroxyéthylammonium.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention peut contenir un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Généralement, le ou les tensioactifs cationiques représentent de 0,1 à 10% en poids du poids total de la composition.

Comme indiqué précédemment, le rapport pondéral tensioactif(s) cationique(s)/amidon(s) est supérieur ou égal à 0,3. De préférence il varie de 0,3 à 10, encore plus préférentiellement de 0,35 à 5, mieux de 0,35 à 1.

La composition selon l'invention peut également comprendre un ou plusieurs agents particulaires additionnels différents des particules creuses de l'invention.

Ces agents particulaires additionnels ont une taille généralement inférieure à 400 µm. Ils peuvent être d'origine naturelle ou synthétique.

Les ou les agents particulaires additionnels sont généralement choisis parmi les poudres végétales, les pigments et les actifs antipelliculaires.

Les pigments peuvent être choisis parmi les pigments organiques ou minéraux.

Parmi les pigments minéraux, on peut citer à titre d'exemple le dioxyde de titane (rutile ou anatase) éventuellement traité en surface et codifié dans le Color Index sous la référence CI 77891 ; les oxydes de fer noir, jaune rouge et brun, codifiés sous les références CI 77499, 77492, 77491 ; le violet de manganèse (CI 77742) ; le bleu outremer (CI 77007) ; l'oxyde de chrome hydraté (CI 77289) ; le bleu ferrique (CI 77510).

Parmi les pigments organiques, on peut citer à titre d'exemple, le pigment YELLOW 3 vendu notamment sous la dénomination commerciale « JAUNE COVANOR W 1603 » par la société WACKHERR (CI 17710), le « D & C RED n°19 » (CI 45170), le « D & C RED n°9 (CI 15585), le « D & C RED n°21 (CI 45380), le « D & C ORANGE n°4 » (CI 15510), le « D & C ORANGE n°5 » (CI 45370), le « D & C RED n°27 » (CI 45410), le « D & C RED n°13 » (CI 15630), le « D & C RED n°7 » (CI 15850-1), le « D & C RED n°6 » (CI 15850-2), le « D & C YELLOW n°5 » (CI 19140), le « D & C RED 36 » (CI 12085), le « D & C ORANGE n°10 » (CI 45425), le « D & C YELLOW n°6 » (CI 15985), le « D & C RED n°30 » (CI 73360), le « D & C RED n°3 » (CI 45430), le « D & C RED n°4 » (CI 14700), le noir de carbone (CI 77266), et les laques à base de carmin de cochenille (CI 75470).

On peut également utiliser des pigments nacrés qui peuvent être notamment choisis parmi les pigments nacrés blancs tels que le mica recouvert d'oxyde de titane, l'oxyde de bismuth ; les pigments nacrés colorés tels que le mica titane avec des oxydes de fer, le mica titane avec du bleu ferrique ou de l'oxyde de chrome, le mica titane avec un pigment organique de type précipité, ainsi que ceux à base d'oxychlorure de bismuth.

On peut également utiliser des pâtes pigmentaires de pigment organique telles que les produits vendus par la société HOECHST sous le nom :

| | |
|---|---|
| JAUNE COSMENYL 10G : | Pigment YELLOW 3 (CI 11710) |
| JAUNE COSMENYL G : | Pigment YELLOW 1 (CI 11680) |
| ORANGE COSMENYL GR : | Pigment ORANGE 43 (CI 71105) |
| ROUGE COSMENYL R° : | Pigment RED 4 (CI 12085) |
| CARMIN COSMENYL FB : | Pigment RED 5 (CI 12490) |
| VIOLET COSMENYL RL : | Pigment VIOLET 23 (CI 51319) |
| BLEU COSMENYL A2R : | Pigment BLUE 15.1 (CI 74260) |
| VERT COSMENYL GG : | Pigment GREEN 7 (CI 74260) |
| NOIR COSMENYL R : | Pigment BLACK 7 (CI 77266) |

A titre de poudre végétales, on peut citer la sciure d'épicéa (extrait de picea excelsea) et la poudre de noyau d'abricot broyée, de préférence à une granulométrie comprise entre 300 et 400 µm.

Généralement, le ou les agents particulaires additionnels différents des particules creuses présentes dans la composition selon l'invention représentent de 0,01 à 30% en poids du poids total de la composition.

La composition selon l'invention peut également comprendre un ou plusieurs corps gras non siliconés.

De préférence, le ou les corps gras non siliconés sont choisis parmi les alcools gras, les acides gras, les esters d'acide gras et d'alcool gras, les cires, les huiles végétales, animales, minérales et synthétiques.

Les alcools gras peuvent être choisis parmi les alcools de formule R'OH, où R' désigne un radical saturé ou insaturé, linéaire ou ramifié, comportant de préférence de 8 à 40 atomes de carbone, de préférence 8 à 30 atomes de carbone. R désigne de préférence un groupement alkyle en C12-C24 ou alkényle en C12-C24. R peut être substitué par un ou plusieurs groupements hydroxy.

Les alcools gras peuvent être en particulier choisis parmi l'alcool laurique, l'alcool cétylique, l'alcool dodécylique, l'alcool décylique, l'alcool stéarylique, l'alcool oléïque, l'alcool béhénique, l'alcool linoléique, l'alcool undécylénique, l'alcool palmitoléïque, l'alcool arachidonique, l'alcool myristylique et l'alcool érucique. On peut également utiliser un mélange d'alcools gras, ce qui signifie que dans un produit commercial peuvent coexister plusieurs espèces d'alcools gras, sous forme d'un mélange. A titre de mélange d'alcools gras, on peut citer l'alcool cétylstéarylique ou cétéarylique.

Les acides gras peuvent être choisis parmi les acides de formule RCOOH, où R est un radical saturé ou insaturé, linéaire ou ramifié, comportant de préférence de 7 à 39 atomes de carbone.

De préférence, R est un groupement alkyle en C7-C29 ou alkényle en C7-C29, mieux un groupement alkyle en C12 - C24 ou alkényle en C12 - C24. R peut être substitué par un ou plusieurs groupements hydroxy et/ou un ou plusieurs groupe carboxyle.

L'acide gras de l'ester peut être en particulier choisi parmi l'acide laurique, l'acide oléique, l'acide palmitique, l'acide linoléique, l'acide myristique et l'acide stéarique.

A titre d'huile végétale, on peut citer l'huile de jojoba, l'huile d'avocat, l'huile de colza, l'huile d'olive, l'huile de tournesol, l'huile de maïs, l'huile de soja, l'huile de courge, l'huile de pépins de raisin, l'huile de sésame, l'huile de noisette, l'huile d'abricot, l'huile de macadamia, l'huile d'arara, l'huile de tournesol, l'huile de ricin.

Le ou les corps gras non siliconés représentent généralement de 0,5 à 20% en poids du poids total de la composition.

La composition selon l'invention peut également comprendre une ou plusieurs silicones.

Les silicones éventuellement présentes dans la composition selon l'invention sont en particulier des polyorganosiloxanes qui peuvent se présenter sous forme de solutions aqueuses, c'est-à-dire solubilisées, ou éventuellement sous forme de dispersions ou micro-dispersions, ou d'émulsions aqueuses. Les polyorganosiloxanes peuvent également se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.

Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.

On peut également citer les cyclocopolymères du type diméthylsiloxanes/méthylalkylsiloxane, tel que la "SILICONE VOLATILE FZ 3109" commercialisée par la société UNION CARBIDE, de structure chimique :
avec D":

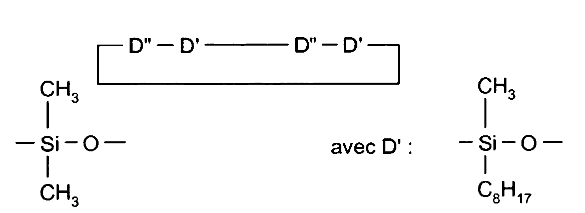

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle (Diméthicone selon la dénomination CTFA) ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

On peut également citer les polydiméthylsiloxanes à groupements aminoéthyl aminopropyl et alpha-oméga silanols.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN 1000 et PH1000 ;
- certaines huiles des séries SF de GENERAL ELECTRIC telles que SF 1023, SF 1154, SF 1250, SF 1265.

Les gommes de silicone pouvant être présentes dans la composition selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane / diphénylsiloxane /méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

Parmi les silicones organomodifiées, on peut encore citer les silicones aminées.

Par silicone aminée, on entend toute silicone comportant au moins une fonction amine primaire, secondaire, tertiaire ou un groupement ammonium quaternaire.

Les silicones aminées éventuellement utilisées dans la composition cosmétique selon la présente invention sont choisies parmi :
(a) les composés répondant à la formule (VI) suivante :

   (R¹)ₐ(T)₃₋ₐ-Si[OSi(T)₂]ₙ-[OSi(T)_{b}(R¹)_{2-b}]ₘ-OSi(T)₃₋ₐ₋(R¹)ₐ (VI)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :

   -N(R²)-CH₂-CH₂-N(R²)₂ ;

   -N(R²)₂ ; -N⁺(R²)₃ Q- ;

   N⁺(R²) (H)2 Q- ;

   N⁺(R²)₂HQ- ;

   -N(R²)-CH₂-CH₂-N⁺(R²)(H)₂ Q-,

   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (VI) sont choisies parmi les composés correspondant à la formule suivante : 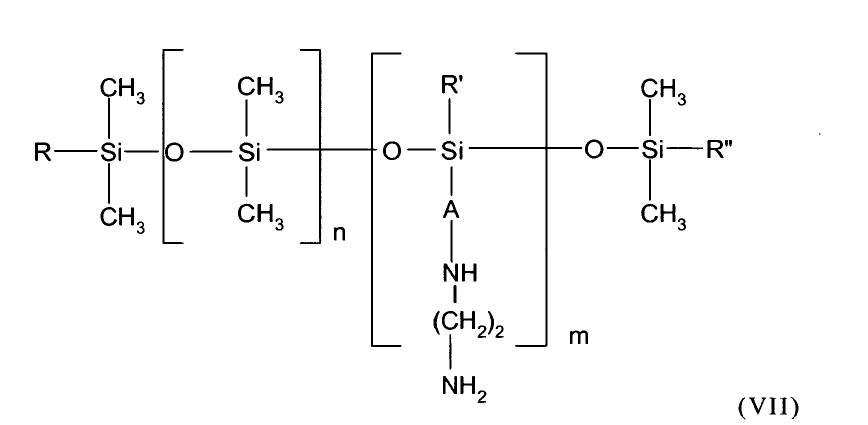 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy /alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (VI) est en particulier le polymère dénommé dans le dictionnaire CTFA "triméthylsilylamodiméthicone", répondant à la formule (VIII) suivante: 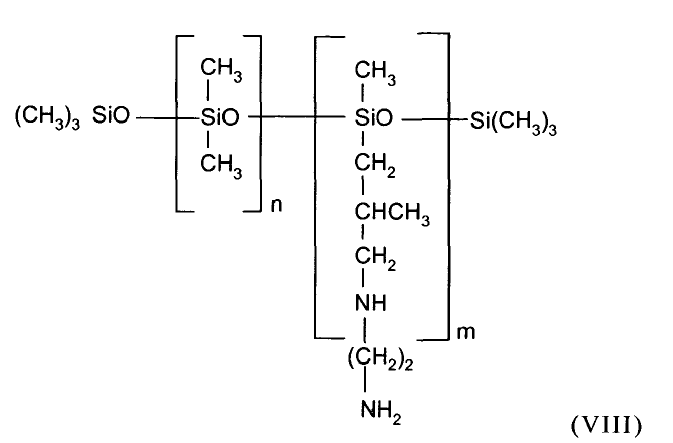 dans laquelle n et m ont les significations données ci-dessus conformément à la formule (VI).

De tels composés sont décrits par exemple dans EP 95238; un composé de formule (VIII) est par exemple vendu sous la dénomination Q2-8220 par la société OSI.

### (b) les composés répondant à la formule (IX) suivante :

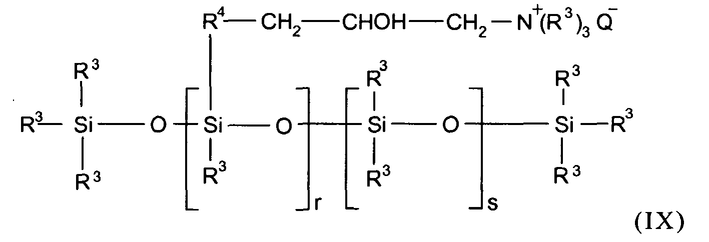

dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈ ;
Q- est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".

### c) les silicones ammonium quaternaire de formule (X) :

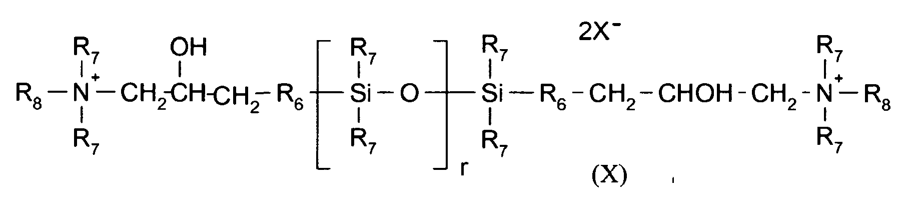

dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;

Ces silicones sont par exemple décrites dans la demande EP-A-0530974.

### d) les silicones aminées de formule (XI) :

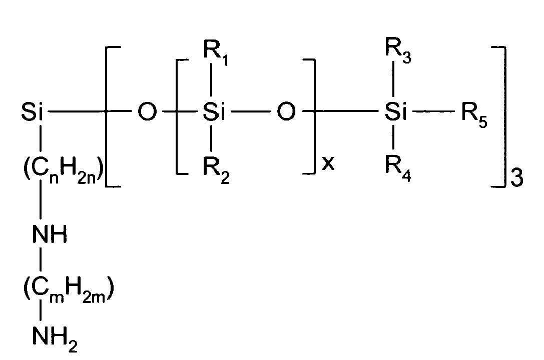

dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

Les silicones particulièrement préférées sont les polydiméthylsiloxanes, les diméthicones et les amodiméthicones.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (VIII) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

La ou les silicones représentent généralement de 0,1 à 20%, de préférence de 0,1 à 10%, en poids du poids total de la composition.

La composition selon l'invention comprend un milieu cosmétiquement acceptable.

Ce milieu est de préférence aqueux, c'est-à-dire qu'il comprend soit uniquement de l'eau, soit de l'eau et un ou plusieurs solvants tels que par exemple l'éthanol, le propylène glycol, le butylène glycol, l'isopropanol, les éthers de glycol tel que les alkyl (C1-C4) éther de mono, di- ou tripropylène glycol, le butylène glycol, l'isopropanol, les éthers de glycol tel que les alkyl(C1-C4) éther de mono, di- ou tripropylène glycol, mono, di- ou triéthylène glycol, le dipropylène glycol, le diéthylène glycol et leurs mélanges.

Le milieu peut être également anhydre ou essentiellement anhydre.

La composition selon l'invention peut en outre comprendre tout additif susceptible d'être utilisé dans le domaine d'application considéré.

Elle est de préférence aqueuse.

En particulier, elle peut comprendre des parfums, des filtres UV, des conservateurs, des antioxydants, des agents régulateurs de pH, des séquestrants, des agents anti-radicaux libres, des hydratants, des agents réducteurs, des agents conditionneurs autres que les silicones et les tensioactifs cationiques tels que des esters, et des vitamines.

L'invention a encore pour objet l'utilisation de la composition telle que définie précédemment pour le conditionnement des cheveux.

L'invention est illustrée par les exemples qui suivent.

### Exemple 1

On a préparé une composition selon l'invention. La formulation est donnée dans le tableau 1. Les teneurs sont exprimées en g de matières actives pour 100g de composition.

**Tableau 1**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,6g |
| Red 4 | 0,003g |
| Acide benzoïque | 0,2g |
| Alcool isopropylique | 0,5g |
| Alcool cétéarylique | 3g |
| Chlorure de béhényl triméthyl ammonium | 2,4g |
| Mélange 96/4 de sel de sodium de carboxyméthylamidon de pomme de terre et d'éthanol (Primojel de la société DMV International) | 7,0g |
| Acide citrique | Qs pH 4 |
| Eau | Qsp 100 g |

### Exemple 2

On a préparé une composition selon l'invention. La formulation est donnée dans le tableau 2. Les teneurs sont exprimées g de matières actives pour 100g de composition.

**Tableau 2**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5.1g |
| Red 4 | 0,003g |
| Acide benzoïque | 0,2g |
| Alcool cétéarylique | 1,5g |
| Mélange 96/4 de sel de sodium de carboxyméthylamidon de pomme de terre et d'éthanol (Primojel de la société DMV International) | 4,4g |
| Méthosulfate de distéaroyléthyl hydroxyéthylammonium | 4,5g |
| Acide citrique | Qs pH 4 |
| Eau | Qsp 100g |

### Exemple 3

On a préparé une composition selon l'invention. La formulation est donnée dans le tableau 3. Les teneurs sont exprimées en g de matières actives pour 100g de composition.

**Tableau 3**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5.1g |
| Red 4 | 0,003g |
| Acide benzoïque | 0,2g |
| Alcool cétéarylique | 4,5g |
| Mélange 96/4 de sel de sodium de carboxyméthylamidon de pomme de terre et d'éthanol (Primojel de la société DMV International) | 4,4 g |
| Méthosulfate de distéaroyléthyl hydroxyéthylammonium | 4,5g |
| Acide citrique | Qs pH 4 |
| Eau | Qsp 100 g |

### Exemple 4

On a préparé une composition selon l'invention. La formulation est donnée dans le tableau 4. Les teneurs sont exprimées g de matières actives pour 100g de composition.

**Tableau 4**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 4,7 |
| Red 4 | 0,003g |
| Acide benzoïque | 0,2g |
| Alcool cétéarylique | 4,2g |
| Huile de graine de jojoba (PURCELL JOJOBA INT) | 1,4g |
| Mélange 96/4 de sel de sodium de carboxyméthylamidon de pomme de terre et d'éthanol (Primojel de la société DMV International) | 7,0 g |
| Méthosulfate de distéaroyléthyl hydroxyéthylammonium | 4,2g |
| Acide citrique | Qs pH 4 |
| Eau | Qsp 100g |

### Exemple 5

On a préparé une composition selon l'invention. La formulation est donnée dans le tableau 5. Les teneurs sont exprimées g de matières actives pour 100g de composition.

**Tableau 5**

| | |
|---|---|
| Parfum | 0,4g |
| Caprylyl Glycol | 0,2g |
| Chlorure de cétyl triméthyl ammonium | 0,03g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5.1g |
| Red 4 | 0,003g |
| Acide benzoïque | 0,2g |
| Alcool cétéarylique | 4,5g |
| Amodiméthicone en emulsion à 60% MA (Dow Corning 939 emulsion de Dow Corning) | 1,7g |
| Tridéceth-6 | 0,14g |
| Mélange 96/4 de sel de sodium de carboxyméthylamidon de pomme de terre et d'éthanol (Primojel de la société DMV International) | 6,4 g |
| Méthosulfate de distéaroyléthyl hydroxyéthylammonium | 4,6g |
| Acide citrique | Qs pH 4 |
| Eau | Qsp 100g |

### Exemple 6

On a préparé une composition selon l'invention. La formulation est donnée dans le tableau 6. Les teneurs sont exprimées en g de matières actives pour 100g de composition.

**Tableau 6**

| | |
|---|---|
| Parfum | 0,4g |
| Caprylyl Glycol | 0,2g |
| Mélange 76/24 de diméthicone et de copolymère réticulé diméthicone/vinyldiméthicone (KSG 16 de la société Shin Etsu) | 3,1 g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 3,4g |
| Red 4 | 0,003g |
| Acide benzoïque | 0,2g |
| Alcool cétéarylique | 4,7g |
| Mélange 96/4 de sel de sodium de carboxyméthylamidon de pomme de terre et d'éthanol (Primojel de la société DMV International) | 5,5 g |
| Méthosulfate de distéaroyléthyl hydroxyéthylammonium | 4,7g |
| Acide citrique | Qs pH 4 |
| Eau | Qsp 100g |

### Exemple 7

On a préparé une composition selon l'invention. La formulation est donnée dans le tableau 7. Les teneurs sont exprimées en g de matières actives pour 100g de composition.

**Tableau 7**

| | |
|---|---|
| Parfum | 0,4 |
| Caprylyl Glycol | 0,2 |
| Yellow 5 | 0,003 |
| Microsphères de copolymère chlorure de vinylidène/acrylonitrile/méthacrylate de méthyle creuses expansées avec isobutane (Expancel 551 DE 40 d42 de la société Expancel) | 5,6g |
| Red 4 | 0,003 g |
| Acide benzoïque | 0,2 g |
| Alcool isopropylique | 0,5 g |
| Alcool cétéarylique | 3 g |
| Chlorure de béhényl triméthyl ammonium | 2,4 g |
| Mélange 96/4 de sel de sodium de carboxyméthylamidon de pomme de terre et d'éthanol (Primojel de la société DMV International) | 7,0 g |
| 3-aminopropyl triéthoxysilane | 3 g |
| Acide citrique | Qs pH 4 |
| Eau | Qsp 100g |

### Exemple 8

On a préparé une composition selon l'invention. La formulation est donnée dans le tableau 8. Les teneurs sont exprimées en g de matières actives pour 100g de composition.

**Tableau 8**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,6g |
| Red 4 | 0,003g |
| Acide citrique | 0g |
| Acide benzoïque | 0,2g |
| Alcool isopropylique | 0,5g |
| Alcool cétéarylique | 3g |
| Chlorure de béhényl triméthyl ammonium | 2,4g |
| Mélange 96/4 de sel de sodium de carboxyméthylamidon de pomme de terre et d'éthanol (Primojel de la société DMV International) | 7,0 g |
| Sciure d'épicéa (extrait de picea excelsa) (farine de bois F140 de SPPS) | 3g |
| Acide citrique | Qs pH 4 |
| Eau | Qsp 100g |

### Exemple 9

On a préparé une composition selon l'invention. La formulation est donnée dans le tableau 9. Les teneurs sont exprimées en g de matières actives pour 100g de composition.

**Tableau 9**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,1g |
| Red 4 | 0,003g |
| Acide benzoïque | 0,2g |
| Alcool cétéarylique | 1,5g |
| Mélange 96/4 de sel de sodium de carboxyméthylamidon de pomme de terre et d'éthanol (Primojel de la société DMV International) | 4,4 g |
| Méthosulfate de distéaroyléthyl hydroxyéthylammonium | 4,5g |
| Sciure d'épicéa (extrait de picea excelsa) (farine de bois F140 de SPPS) | 1,9g |
| Acide citrique | Qs pH 4 |
| Eau | Qsp 100g |

### Exemple 10

On a préparé une composition selon l'invention. La formulation est donnée dans le tableau 10. Les teneurs sont exprimées en pourcentages en poids g de matières actives pour 100g de composition.

**Tableau 10**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,1g |
| Red 4 | 0,003g |
| Acide benzoïque | 0,2g |
| Alcool cétéarylique | 1,5g |
| Poudre de graines de Prunus armenica (Abricot) | 10g |
| Mélange 96/4 de sel de sodium de carboxyméthylamidon de pomme de terre et d'éthanol (Primojel de la société DMV International) | 4,4 g |
| Méthosulfate de distéaroyléthyl hydroxyéthylammonium | 4,5g |
| Acide citrique | Qs pH 4 |
| Eau | Qsp 100g |

### Exemple 11

On a préparé une composition selon l'invention. La formulation est donnée dans le tableau 11. Les teneurs sont exprimées en g de matières actives pour 100g de composition.

**Tableau 11**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,1g |
| Red 4 | 0,003g |
| Acide benzoïque | 0,2g |
| Alcool cétéarylique | 4,3g |
| Mélange 96/4 de sel de sodium de carboxyméthylamidon de pomme de terre et d'éthanol (Primojel de la société DMV International) | 7,0 g |
| Méthosulfate de distéaroyléthyl hydroxyéthylammonium | 4,3g |
| Sciure d'épicéa (extrait de picea excelsa) (farine de bois F140 de SPPS) | 3g |
| Acide citrique | Qs pH 4 |
| Eau | Qsp 100g |

### Exemple 12

On a préparé une composition selon l'invention. La formulation est donnée dans le tableau 12. Les teneurs sont exprimées en g de matières actives pour 100g de composition.

**Tableau 12**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,1g |
| Red 4 | 0,003g |
| Acide benzoïque | 0,2g |
| Alcool cétéarylique | 4,4g |
| Huile de graine de jojoba | 1,5g |
| Mélange 96/4 de sel de sodium de carboxyméthylamidon de pomme de terre et d'éthanol (Primojel de la société DMV International) | 4,4 g |
| Méthosulfate de distéaroyléthyl hydroxyéthylammonium | 4,4g |
| Sciure d'épicéa (extrait de picea excelsa) (farine de bois F140 de SPPS) | 1,9g |
| Acide citrique | Qs pH 4 |
| Eau | Qsp 100g |

### Exemple 13

On a préparé une composition selon l'invention. La formulation est donnée dans le tableau 13. Les teneurs sont exprimées en g de matières actives pour 100g de composition.

**Tableau 13**

| | |
|---|---|
| Parfum | 0,4g |
| Caprylyl Glycol | 0,2g |
| Chlorure de cétyl triméthyl ammonium | 0,03g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,1g |
| Red 4 | 0,003g |
| Acide benzoïque | 0,2g |
| Alcool cétéarylique | 4,5g |
| Amodiméthicone | 1,7g |
| Tridiceth-6 | 0,2g |
| Mélange 96/4 de sel de sodium de carboxyméthylamidon de pomme de terre et d'éthanol (Primojel de la société DMV International) | 6,4g |
| Méthosulfate de distéaroyléthyl hydroxyéthylammonium | 4,6g |
| Sciure d'épicéa (extrait de picea excelsa) | 2,7g |
| Acide citrique | Qs pH 4 |
| Eau | Qsp 100g |

### Exemple 14

On a préparé une composition selon l'invention. La formulation est donnée dans le tableau 14. Les teneurs sont exprimées en g de matières actives pour 100g de composition.

**Tableau 14**

| | |
|---|---|
| Parfum | 0,4g |
| Caprylyl Glycol | 0,2g |
| Mélange 76/24 de diméthicone et de copolymère réticulé diméthicone/vinyldiméthicone (KSG 16 de la société Shin Etsu) | 3,1g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 3,4g |
| Red 4 | 0,003g |
| Acide benzoïque | 0,2g |
| Alcool cétéarylique | 4,7g |
| Mélange 96/4 de sel de sodium de carboxyméthylamidon de pomme de terre et d'éthanol (Primojel de la société DMV International) | 5,5g |
| Méthosulfate de distéaroyléthyl hydroxyéthylammonium | 4,7g |
| Sciure d'épicéa (extrait de picea excelsa) | 2,34g |
| Acide citrique | Qs pH 4 |
| Eau | Qsp 100g |

### Exemple 15

On a préparé une composition selon l'invention. La formulation est donnée dans le tableau 15. Les teneurs sont exprimées en g de matières actives pour 100g de composition.

**Tableau 15**

| | |
|---|---|
| Parfum | 0,4g |
| Caprylyl Glycol | 0,2g |
| Yellow 5 | 0,003g |
| Microsphères de copolymère chlorure de vinylidène/acrylonitrile/méthacrylate de méthyle creuses expansées avec isobutane (Expancel 551 DE 40 d42 de la société Expancel) | 5,6g |
| Red 4 | 0,003g |
| Acide benzoïque | 0,2g |
| Alcool isopropylique | 0,5g |
| Alcool cétéarylique | 3g |
| Chlorure de béhényl triméthyl ammonium | 2,3g |
| Mélange 96/4 de sel de sodium de carboxyméthylamidon de pomme de terre et d'éthanol (Primojel de la société DMV International) | 7,0 g |
| Sciure d'épicéa (extrait de picea excelsa) | 3g |
| Triéthoxysilane aminopropyl | 3g |
| Acide citrique | Qs pH 4 |
| Eau | Qsp 100g |

Les compositions des exemples 1 à 15 sont des soins capillaires à rincer sous forme de solides déformables qui ont de très bonnes performances cosmétiques en apportant une grande facilité de démêlage et une grande douceur aux cheveux, en particulier normaux à secs. On constate également un bon effet coiffant. Les qualités d'usage sont bonnes : une quantité de produit assez faible en poids suffit en général à traiter toute la chevelure. L'application sur cheveux est facile, le rinçage est très aisé et rapide.

### Exemple 16

On a comparé une composition selon l'invention (composition B) ayant un rapport pondéral tensioactif(s) cationique(s)/amidon(s) supérieur à 0,3 avec une composition comparative (composition A) ayant un rapport pondéral tensioactif(s) cationique(s)/amidon(s) inférieur à 0,3.

La composition B selon l'invention et la composition A hors invention ont été préparées selon les formulations données dans le tableau 16. Les teneurs sont exprimées en g de matières actives pour 100g de composition.

**Tableau 16**

| | A | B |
|---|---|---|
| Carboxyméthylamidon de pomme de terre réticulé (Primojel de la société DMV International) | 27 | 20,7 |
| Farine de bois d'épicéa (farine de bois F140 de SPPS) | 22 | 22 |
| Microsphère expansées de copolymère acrylonitrile / méthacrylate de méthyle (Expancel 551 DE 40 d42 de la société AKZO NOBEL) | 5 | 5 |
| Propylène glycol | 16 | 16 |
| Chlorure de cétyl triméthyl ammonium (GENAMIN CTAC 50 ET de la société CLARIANT) | 4 | 10,3 |
| Copolymère de chlorure de diméthyldiallyl ammonium / acide acrylique (MERQUAT 280 de la société NALCO) | 2,7 | 2,7 |
| Eau | Qs 100 | Qs 100 |
| Rapport TA cationique / amidon | 0,15 | 0,5 |

La texture de la composition A est très sèche et très friable. Sous l'eau, elle se délite moyennement bien (il reste des amas dans les mains), et conduit à une texture peu onctueuse après transformation en crème dans la main.

La composition B est moins sèche et moins friable. Elle se délite nettement mieux que la composition A, et conduit à une texture plus onctueuse après transformation en crème dans la main.

Les compositions A et B ont été appliquées par demi-tête sur une tête malléable, à raison de 6g de composition par demi-tête puis rinçées à l'eau.

7 experts ont évalué les propriétés sur une échelle de 0 (pas bon) à 5 (très bon).

Le tableau 17 rassemble les moyennes des notes obtenues sur les critères évalués.

**Tableau 17**

| | A | B |
|---|---|---|
| Fondant à l'application | 0,8 | 2,1 |
| Souplesse des cheveux à l'application | 1,1 | 2,4 |
| Souplesse des cheveux au rinçage | 0,8 | 2,2 |
| Démêlage des cheveux humides | 1,5 | 2,5 |
| Souplesse des cheveux humides | 1,2 | 2,1 |

La composition B selon l'invention présente des propriétés cosmétiques nettement supérieures à celles la composition A.

### Exemple 17

On a comparé une composition selon l'invention (composition C) ayant un rapport pondéral tensioactif(s) cationique(s)/amidon(s) supérieur à 0,3 avec le composition comparative de l'exemple 16 (composition A) ayant un rapport pondéral tensioactif(s) cationique(s)/amidon(s) inférieur à 0,3.

La composition C selon l'invention et la composition A hors invention ont été préparées selon les formulations données dans le tableau 18. Les teneurs sont exprimées en g de matières actives pour 100g de composition.

**Tableau 18**

| | A | C |
|---|---|---|
| Carboxyméthylamidon de pomme de terre réticulé (Primojel de la société DMV International) | 27 | 8 |
| Farine de bois d'épicéa (farine de bois F140 de SPPS) | 22 | 22 |
| Microsphère expansées de copolymère acrylonitrile / méthacrylate de méthyle (Expancel 551 DE 40 d42 de la société AKZO NOBEL) | 5 | 5 |
| Propylène glycol | 16 | 16 |
| Chlorure de cétyl triméthyl ammonium (GENAMIN CTAC 50 ET de la société CLARIANT) | 4 | 4 |
| Copolymère de chlorure de diméthyldiallyl ammonium / acide acrylique (MERQUAT 280 de la société NALCO) | 2,7 | 2,7 |
| Eau | Qs 100 | Qs 100 |
| Rapport TA cationique / amidon | 0,15 | 0,5 |

La texture de la composition A est très sèche et très friable. Sous l'eau, elle se délite moyennement bien (il reste des amas dans les mains), et conduit à une texture peu onctueuse après transformation en crème dans la main.

La composition C n'est pas sèche et n'est pas friable Elle présente un très bon délitement et conduit à une texture très onctueuse après transformation en crème dans la main.

Les compositions A et C ont été appliquées par demi-tête sur une tête malléable, à raison de 6g de composition par demi-tête, puis ont été rincées à l'eau.

6 experts ont évalué les propriétés sur une échelle de 0 (pas bon) à 5 (très bon).

Le tableau 19 rassemble les moyennes des notes obtenues sur les critères évalués.

**Tableau 19**

| | A | C |
|---|---|---|
| Fondant à l'application | 0,8 | 2 |
| Lissage au toucher des cheveux secs | 2,5 | 3 |

La composition C selon l'invention présente des propriétés cosmétiques nettement supérieures à la composition A.

## Revendications

1. Composition cosmétique se présentant sous forme de solide déformable comprenant, dans un milieu cosmétiquement acceptable, un ou plusieurs types de particules creuses, un ou plusieurs amidons modifiés ou non, et un ou plusieurs tensioactifs cationiques, le rapport pondéral tensioactif(s) cationique(s)/amidon(s) étant supérieur ou égal à 0,3.

2. Composition selon la revendication 1 **caractérisée en ce que** le rapport pondéral tensioactif(s) cationique(s)/amidon(s) varie de 0,3 à 10, encore plus préférentiellement de 0,35 à 5, mieux de 0,35 à 1.

3. Composition selon les revendications 1 ou 2 **caractérisée en ce que** les particules creuses sont choisies parmi les particules de verre, les particules de matériaux thermoplastiques choisis parmi les polyamides, les polymères ou copolymères d'acrylonitrile, de chlorure de vinylidène, de chlorure de vinyle et/ou de monomère acrylique ou styrénique, éventuellement expansés, les microsphères microporeuses et les microparticules de silice.

4. Composition selon la revendication 3 **caractérisée en ce que** les particules creuses sont choisies parmi les particules creuses d'un copolymère expansé de chlorure de vinylidène et d'acrylonitrile, ou de chlorure de vinylidène, d'acrylonitrile et de méthacrylate de méthyle.

5. Composition selon l'une quelconque des revendications précédentes **caractérisée en ce que** les particules creuses représentent de 0,01 à 15%, de préférence de 1 à 10%, en poids du poids total de la composition.

6. Composition selon l'une quelconque des revendications précédentes **caractérisée en ce que** le ou lesdits amidons proviennent d'une source végétale choisie parmi le maïs, les pois, la pomme de terre, la patate douce, la banane, l'orge, le blé, le riz, l'avoine, le sagou, le tapioca et le sorgo.

7. Composition selon l'une quelconque des revendications précédentes **caractérisée en ce que** le ou lesdits amidons modifiés sont choisis parmi le phosphate de diamidon hydroxypropylé, l'amidon modifié par l'acide 2-chloroéthyl aminodipropionique et les carboxyméthylamidons.

8. Composition selon l'une quelconque des revendications précédentes **caractérisé en ce que** le ou les amidons représentent de 0,01 à 15%, de préférence de 0,1 à 10% mieux de 1 à 10%, en poids du poids total de la composition.

9. Composition selon l'une quelconque des revendications précédentes **caractérisée en ce que** le ou les tensioactifs cationiques représentent de 0,1 à 10% en poids du poids total de la composition.

10. Composition selon l'une quelconque des revendications précédentes **caractérisée en ce qu'**elle comprend un ou plusieurs agents particulaires additionnels autre que lesdites particules creuses.

11. Composition selon la revendication 10 **caractérisée en ce que** le ou les agents particulaires additionnels sont choisis parmi les poudres végétales, les pigments et les actifs antipelliculaires.

12. Composition selon la revendication 10 ou 11 **caractérisée en ce que** le ou les agents particulaires additionnels représentent de 0,01 à 30% en poids du poids total de la composition.

13. Composition selon l'une quelconque des revendications précédentes **caractérisée en ce qu'**elle comprend un ou plusieurs corps gras non siliconés.

14. Composition selon l'une quelconque des revendications précédentes **caractérisée en ce qu'**elle comprend une ou plusieurs silicones.

15. Utilisation de la composition telle que définie dans l'une quelconque des revendications précédentes pour le conditionnement des cheveux.

## Claims

1. Cosmetic composition in the form of a deformable solid comprising, in a cosmetically acceptable medium, one or more types of hollow particles, one or more modified or unmodified starches, and one or more cationic surfactants, the weight ratio of cationic surfactant(s) to starch or starches being greater than or equal to 0.3.

2. Composition according to Claim 1, **characterized in that** the weight ratio of cationic surfactant(s) to starch or starches varies from 0.3 to 10, more preferably from 0.35 to 5, and even more preferably from 0.35 to 1.

3. Composition according to Claims 1 or 2, **characterized in that** the hollow particles are selected from glass particles, particles of thermoplastic materials selected from polyamides, polymers or copolymers of acrylonitrile, of vinylidene chloride, of vinyl chloride and/or of acrylic or styrene monomer, optionally expanded, microporous microspheres and silica microparticles.

4. Composition according to Claim 3, **characterized in that** the hollow particles are selected from hollow particles of an expanded copolymer of vinylidene chloride and acrylonitrile, or of vinylidene chloride, acrylonitrile and methyl methacrylate.

5. Composition according to any one of the preceding claims, **characterized in that** the hollow particles represent from 0.01 to 15 wt.%, preferably from 1 to 10 wt.%, of the total weight of the composition.

6. Composition according to any one of the preceding claims, **characterized in that** said starch or starches are from a vegetable source selected from maize, peas, potato, sweet potato, banana, barley, wheat, rice, oat, sago, tapioca and sorghum.

7. Composition according to any ono of the preceding claims, **characterized in that** said modified starch or starches are selected from hydroxypropylated distarch phosphate, starch modified with 2-chloroethylaminopropionic acid and carboxymethyl-starches.

8. Composition according to any one of the preceding claims, **characterized in that** the starch or starches represent from 0.01 to 15 wt.%, preferably from 0.1 to 10 wt.%, more preferably from 1 to 10 wt.%, of the total weight of the composition.

9. Composition according to any one of the preceding claims, **characterized in that** the cationic surfactant or surfactants represent from 0.1 to 10 wt.% of the total weight of the composition.

10. Composition according to any one of the preceding claims, **characterized in that** it comprises one or more additional particulate agents other than said hollow particles.

11. Composition according to Claim 10, **characterized in that** the additional particulate agent or agents are selected from vegetable powders, pigments and antidandruff agents.

12. Composition according to Claim 10 or 11, **characterized in that** the additional particulate agent or agents represent from 0.01 to 30 wt.% of the total weight of the composition.

13. Composition according to any one of the preceding claims, **characterized in that** it comprises one or more non-silicone fats.

14. Composition according to any one of the preceding claims, **characterized in that** it comprises one or more silicones.

15. Use of the composition as defined in any one of the preceding claims for conditioning the hair.

## Patentansprüche

1. Kosmetische Zusammensetzung, die in Form eines verformbaren Feststoffs vorliegt und in einem kosmetisch unbedenklichen Medium einen oder mehrere Typen von Hohlpartikeln, eine oder mehrere gegebenenfalls modifizierte Stärken und ein oder mehrere anionische Tenside umfasst, wobei das Gewichtsverhältnis von anionischem Tensid bzw. anionischen Tensiden zu Stärke bzw. Stärken größer gleich 0,3 ist.

2. Zusammensetzung nach Anspruch 1, **dadurch gekennzeichnet, dass** das Gewichtsverhältnis von anionischem Tensid bzw. anionischen Tensiden zu Stärke bzw. Stärken von 0,3 bis 10, weiter bevorzugt 0,35 bis 5 und noch weiter bevorzugt 0,35 bis 1 variiert.

3. Zusammensetzung nach den Ansprüche 1 oder 2, **dadurch gekennzeichnet, dass** die Hohlpartikel unter Glaspartikeln, Partikeln aus thermoplastischen Materialien, die unter Polyamiden, Polymeren oder Copolymeren von Acrylnitril, Vinylidenchlorid, Vinylchlorid und/oder Acryl- oder Styrolmonomer, die gegebenenfalls expandiert sind, mikroporösen Mikrokugeln und Mikropartikeln aus Siliciumdioxid ausgewählt sind.

4. Zusammensetzung nach Anspruch 3, **dadurch gekennzeichnet, dass** die Hohlpartikel aus einem expandierten Copolymer von Vinylidenchlorid und Acrylnitril oder Vinylidenchlorid, Acrylnitril und Methylmethacrylat ausgewählt sind.

5. Zusammensetzung nach einem der vorhergehenden Ansprüche, **dadurch gekennzeichnet, dass** die Hohlpartikel 0,01 bis 15 Gew.-% und vorzugsweise 1 bis 10 Gew.-% des Gesamtgewichts der Zusammensetzung ausmachen.

6. Zusammensetzung nach einem der vorhergehenden Ansprüche, **dadurch gekennzeichnet, dass** die Stärke bzw. die Stärken aus einer pflanzlichen Quelle, die unter Mais, Erbsen, Kartoffeln, Süßkartoffeln, Bananen, Gerste, Weizen, Reis, Hafer, Sago, Tapioka und Sorghum ausgewählt ist, stammt bzw. stammen.

7. Zusammensetzung nach einem der vorhergehenden Ansprüche, **dadurch gekennzeichnet, dass** die modifizierte Stärke bzw. die modifizierten Stärken unter Hydroxypropyldistärkephosphat, mit 2-Chlorethylaminodipropionsäure modifizierter Stärke und Carboxymethylstärken ausgewählt ist bzw. sind.

8. Zusammensetzung nach einem der vorhergehenden Ansprüche, **dadurch gekennzeichnet, dass** die Stärke bzw. die Stärken 0,01 bis 15 Gew.-%, vorzugsweise 0,1 bis 10 Gew.-% und weiter bevorzugt 1 bis 10 Gew.-% des Gesamtgewichts der Zusammensetzung ausmacht bzw. ausmachen.

9. Zusammensetzung nach einem der vorhergehenden Ansprüche, **dadurch gekennzeichnet, dass** das kationische Tensid bzw. die kationischen Tenside 0,1 bis 10 Gew.-% des Gesamtgewichts der Zusammensetzung ausmacht bzw. ausmachen.

10. Zusammensetzung nach einem der vorhergehenden Ansprüche, **dadurch gekennzeichnet, dass** sie neben den Hohlpartikeln noch ein oder mehrere zusätzliche spezielle Agenzien umfasst.

11. Zusammensetzung nach Anspruch 10, **dadurch gekennzeichnet, dass** das zusätzliche spezielle Agens bzw. die zusätzlichen speziellen Agenzien unter pflanzlichen Pulvern, Pigmenten und Antischuppenmitteln ausgewählt ist bzw. sind.

12. Zusammensetzung nach Anspruch 10 oder 11, **dadurch gekennzeichnet, dass** das zusätzliche spezielle Agens bzw. die zusätzlichen speziellen Agenzien 0,01 bis 30 Gew.-% des Gesamtgewichts der Zusammensetzung ausmacht bzw. ausmachen.

13. Zusammensetzung nach einem der vorhergehenden Ansprüche, **dadurch gekennzeichnet, dass** sie eine oder mehrere Nichtsilikon-Fettsubstanzen umfasst.

14. Zusammensetzung nach einem der vorhergehenden Ansprüche, **dadurch gekennzeichnet, dass** sie ein oder mehrere Silikone umfasst.

15. Verwendung der Zusammensetzung gemäß einem der vorhergehenden Ansprüche zur Konditionierung der Haare.
